# EUROPEAN PATENT APPLICATION

(11) **EP 3 760 640 A1**
(43) Date of publication of application: **06.01.2021**
(21) Application number: 19760318.6
(22) Date of filing: 22.02.2019
(51) Int. Cl.: C07K 16/18, G01N 33/531, G01N 33/543

(54) **ANTIBODY THAT SPECIFICALLY RECOGNIZES N TERMINUS OF APP669-X, AND IMMUNOASSAY METHOD**

(30) Priority: 27.02.2018 WO PCT/JP2018/007295; 13.09.2018 JP 2018171278
(71) Applicant: Shimadzu Corporation, Kyoto-shi Kyoto 604-8511 (JP)
(72) Inventor: KANEKO, Naoki, Kyoto-shi, Kyoto 604-8511 (JP)
(74) Representative: Mewburn Ellis LLP
(86) International application number: PCT/JP2019/006778
(87) International publication number: WO 2019/167830

(57) **Abstract**

The present invention provides an antibody that recognizes peptides the N-terminals of which start from APP669 (collectively referred to as APP669-x) including the peptide APP669-711 related to an amyloid β (Aβ) ; a method for measuring APP669-x using said antibody; and a sandwich immunoassay method that is capable of detecting and quantifying a polypeptide to be analyzed with a high sensitivity. An APP669-x N-terminal-recognizing monoclonal antibody that specifically recognizes an N-terminal of an APP669-x peptide. An immunoassay method that uses an APP669-x N-terminal-recognizing monoclonal antibody that specifically recognizes an N-terminal of an APP669-x peptide, and comprises reacting an APP669-x in a sample with the APP669-x N-terminal-recognizing monoclonal antibody to measure the APP669-x.

SEQUENCE LISTING

<110>  SHIMADZU CORPORATION

<120>  Antibody Specifically Recognizing N-termimus Of APP669-x And
Immunoassay Method

<130>  SP20180164

<150>  WO PCT/JP2018/007295
<151>  2018-02-27

<150>  JP 2018-171278
<151>  2018-09-13

<160>  9

<210>  1
<211>  40
<212>  PRT
<213>  Homo Sapiens

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  2
<211>  42
<212>  PRT
<213>  Homo Sapiens

<400>  2

<210>  3
<211>  43
<212>  PRT
<213>  Homo Sapiens

<400>  3

Val Lys Met Asp Ala Glu Phe Arg His Asp Ser Gly Tyr Glu Val His
1               5                   10                  15

His Gln Lys Leu Val Phe Phe Ala Glu Asp Val Gly Ser Asn Lys Gly
            20                  25                  30

Ala Ile Ile Gly Leu Met Val Gly Gly Val Val
        35                  40

<210>  4
<211>  10
<212>  PRT
<213>  Homo Sapiens

<400>  4

Glu Val Lys Met Asp Ala Glu Phe Arg His
1               5                   10

<210>  5
<211>  4
<212>  PRT
<213>  Homo Sapiens

<400>  5

Val Lys Met Cys
1

<210>  6
<211>  38
<212>  PRT
<213>  Homo Sapiens

<400>  6

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly
        35

<210>  7
<211>  38
<212>  PRT
<213>  Homo Sapiens

<400>  7

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val
        35

<210>  8
<211>  39
<212>  PRT
<213>  Homo Sapiens

<400>  8

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val
        35

<210>  9
<211>  40
<212>  PRT
<213>  Homo Sapiens

<400>  9

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

## Description

### TECHNICAL FIELD

The present invention relates to an immunoassay method in the Alzheimer's disease field. More specifically, the present invention relates to an antibody that recognizes peptides the N-terminals of which start from APP669 (collectively referred to as APP669-x) including the peptide APP669-711 related to an amyloid β (Aβ), and a method for measuring APP669-x using the antibody.

The present invention pertains to the field of the clinical test reagent for a trace component, an infectious microorganism antigen, and so on; the biochemistry research field; the immunology research field; and the like, and the present invention also relates to a sandwich immunoassay method that utilizes a specific immunoreaction. More specifically, the present invention also relates to a sandwich immunoassay method that is useful for detecting and quantifying a trace amount of polypeptide in a sample.

### BACKGROUND ART

Alzheimer's disease (AD) is a principal cause of dementia, and occupies 50 to 60% of the entire dementia. The number of patients suffering from dementia was more than or equal to 24 million in the world in 2001, and is estimated to reach 81 million in 2040. It is considered that an Aβ is deeply involved in development of Alzheimer's disease. The Aβ is produced as a result of proteolysis of amyloid precursor protein (APP) which is a single-pass transmembrane protein composed of 770 amino acid residues, by β secretase and γ secretase. Appearance of senile plaques due to aggregation of Aβ accompanying fibrosis triggers aggregation and accumulation of tau protein inside neurocytes to cause nerve malfunction and neuronal cell death. It is considered that this results in extreme deterioration of the cognitive ability. It has long been known that Aβ mainly consists of 40 mer (Aβ1-40) and 42 mer (Aβ1-42), and migrates into cerebrospinal fluid (CSF). Furthermore, it is suggested that there is some possibility that Aβ also migrates into blood. Further, in recent years, existence of Aβ-like peptides having lengths different from those of Aβ1-40 and Aβ1-42 in CSF has been reported.

As for Aβ, the present inventor et al. have reported in the recent research that the ratio of APP669-711 which is one of Aβ related peptides (Aβ-like peptides) to Aβ1-42 is promising as a blood biomarker (Non-Patent Document 1, Patent Document 1: WO 2015/178398). These Aβ and Aβ related peptides are quantified by an immunoprecipitation method (IP) followed by a mass spectrometry (MALDI-TOF MS).

Furthermore, the present inventor et al. have disclosed in Patent Document 2: WO 2017/047529 that a numerical value by a combination of two or more ratios selected from the group consisting of three ratios, Aβ1-39/Aβ1-42, Aβ1-40/Aβ1-42, and APP669-711/Aβ1-42, regarding Aβ and Aβ related peptides (Aβ-like peptides), through a mathematical technique is promising as a blood biomarker. These Aβ and Aβ related peptides are quantified by an immunoprecipitation method (IP) followed by a mass spectrometry (MALDI-TOF MS).

As mentioned above, the present inventor et al. have discovered by using a mass spectrometry (MALDI-TOF MS) that a ratio between APP669-711 which is one of Aβ related peptides and Aβ1-42 is promising as a candidate for a blood biomarker. Furthermore, the present inventor et al. have reported that a composite biomarker by a combination of the ratio of APP669-711/Aβ1-42 and the ratio of Aβ1-40/Aβ1-42 can estimate a cerebral amyloid accumulation with high accuracy through the use of multiple specimens in Japan and Australia, accordingly, the composite biomarker is a reliable and versatile biomarker (Non-Patent Document 2).

A mass spectrometry (MALDI-TOF MS) is useful as an analysis technique for biomarkers such as Aβ related peptides including APP669-711. Meanwhile, as the other analysis technique, a sandwich ELISA is a widely and generally used measurement method as a clinical examination method and is low cost method. Accordingly, the sandwich ELISA can be a useful analysis technique.

At present, an ELISA Kit capable of analyzing Aβ1-40 and Aβ1-42 in the plasma is commercially available from respective makers (Wako Pure Chemical Corporation, IBL, etc.).

Patent Document 3: JP-A-2005-170951 discloses the monoclonal antibodies BAN-52a and BAN-50a that recognize the N-terminal portion of an amyloid β (Aβ1-16), and the monoclonal antibodies BA-27a, BS-85 and BC-05a that specifically recognize the C-terminal portion of an amyloid β, and discloses a sandwich EIA which is specific to Aβ1-40 and Aβ1-42.

Patent Document 4: JP-A-2014-208678 discloses an antibody that specifically recognizes Aβ11-x, and discloses a sandwich ELISA to Aβ11-40 and a western blotting to Aβ11-x.

However, an analytical means for APP669-x using an immunoassay method is not known.

An immunoassay method is a method of measuring a concentration of a substance to be analyzed in a sample, utilizing a specific antigen-antibody reaction. The immunoassay method is capable of measuring a trace component specifically and accurately, accordingly, is widely used in the clinical test, the biochemistry research, and the like.

The immunoassay method includes an enzyme immunoassay method (EIA) utilizing an enzyme as a labeling substance, a radio immunoassay method (RIA) utilizing a radioisotope, a chemiluminescent immunoassay method (CIA) utilizing a chemiluminescent substance, a fluorescent immunoassay method (FIA) utilizing a fluorescent substance, an electro-chemiluminescent immunoassay method (ECLIA) utilizing a metal complex, a bioluminescent immunoassay method (BLIA) utilizing a bioluminescent substance such as a luciferase, an immuno-PCR comprising amplifying a nucleic acid that labeled an antibody by PCR and detecting the nucleic acid, a turbidimetric immunoassay method (TAI) detecting turbidity occurred by forming an immunocomplex, a latex agglutination turbidimetric method (LA) detecting a latex aggregated by forming an immunocomplex, an immunochromatography assay utilizing a reaction on a cellulose membrane, and the like.

Among the immunoassay methods, a method wherein two antibodies that recognize different epitopes from each other of a substance to be analyzed are used to sandwich the substance to be analyzed is referred to as a sandwich method. For example, a sandwich ELISA (Enzyme-Linked ImmunoSorbent Assay) is one of EIA, and is a method wherein a substance to be analyzed is captured by a capture antibody immobilized on a surface of 96 well plate, the substance to be analyzed is reacted with a detection antibody labeled with an enzyme such as a peroxidase, and an alkaline phosphatase, and a concentration of the substance to be analyzed is determined by utilizing coloration of a substrate of the labeling enzyme (for example, Patent Document 5: JP-A-1996-220098). The ECLIA also utilizes a sandwich method wherein a substance to be analyzed is reacted with a biotinylated antibody and an antibody labeled with a ruthenium (Ru) complex that emits light depending on an electrochemical change (for example, Patent Document 6: JP-A-2014-509735). Thus, the sandwich method is widely utilized in the immunoassay methods.

The sandwich method is also utilized as a method of determining specifically the quantity of a specific peptide fragment. That is, a peptide fragment to be analyzed is sandwiched with an antibody that recognizes the N-terminal of the peptide fragment and an antibody that recognizes the C-terminal of the peptide fragment, to measure specifically the peptide fragment. For example, in respect of an Aβ which is produced by cleavage of amyloid precursor protein (APP) by protease, various Aβ peptide fragments are exist in a living body. Among them, Aβ1-42 is, as a cerebrospinal fluid (CSF) biomarker for the Alzheimer's disease, quantified by an immunoassay method such as the ELISA (Non-Patent Document 3) .

Patent Document 7: JP-A-2004-239885 reports, as one way improving a sensitivity of an immunoassay method, a method extracting a sample protein by using high concentration of an ionic surfactant before conducting the immunoassay method, in a case where the protein is hard to be extracted from the sample.

### PRIOR ART DOCUMENTS

### PATENT DOCUMENTS

Patent Document 1: WO 2015/178398
Patent Document 2: WO 2017/047529
Patent Document 3: JP-A-2005-170951
Patent Document 4: JP-A-2014-208678
Patent Document 5: JP-A-1996-220098
Patent Document 6: JP-A-2014-509735
Patent Document 7: JP-A-2004-239885

### NON-PATENT DOCUMENTS

Non-Patent Document 1: Kaneko N, Nakamura A, Washimi Y, Kato T, Sakurai T, Arahata Y, Bundo M, Takeda A, Niida S, Ito K, Toba K, Tanaka K, Yanagisawa K. : Novel plasma biomarker surrogating cerebral amyloid deposition. Proc Jpn Acad Ser B Phys Biol Sci. 2014; 90(9): 353-64.

Non-Patent Document 2: Nakamura A, Kaneko N, Villemagne VL, Kato T, Doecke J, Doré V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K. : High performance plasma amyloid-β biomarkers for Alzheimer's disease. Nature. 2018; 554 (7691): 249-254.

Non-Patent Document 3: Blennow K. : Cerebrospinal Fluid Protein Biomarkers for Alzheimer's Disease. The Journal of the American Society for Experimental NeuroTherapeutics 2004 Apr;1(2):213-25.

Non-Patent Document 4: Murakami K, Masuda Y, Shirasawa T, Shimizu T, Irie K. : The turn formation at positions 22 and 23 in the 42-mer amyloid beta peptide: the emerging role in the pathogenesis of Alzheimer's disease. Geriatr Gerontol Int. 2010 Jul;10 Suppl 1: S169-79.

### SUMMARY OF THE INVENTION

### PROBLEMS TO BE SOLVED BY THE INVENTION

In order to construct a sandwich ELISA specific to a peptide, it will be in need of respective antibodies that specifically recognizes N-terminal and C-terminal of the peptide. C-terminal of APP669-711 is the same as C-terminal of Aβ1-40 (namely, APP672-711), accordingly, there exists an antibody that specifically recognizes C-terminal of APP669-711. However, an antibody that specifically recognizes N-terminal of APP669-711 is not exist.

Since an antibody that specifically recognizes N-terminal of APP669-x is not exist, there are no means for a sandwich ELISA specifically quantifying APP669-711, and an immunoassay method analyzing APP669-x.

Therefore, an object of the present invention is to provide an antibody that recognizes peptides the N-terminals of which start from APP669 (collectively referred to as APP669-x) including the peptide APP669-711 related to an amyloid β (Aβ). Further, an object of the present invention is to provide a method for measuring APP669-x using said antibody.

In order to specifically quantify a polypeptide to be analyzed in a sample by a sandwich immunoassay method, toward two different sites (epitopes) of the polypeptide to be analyzed, two antibodies that recognize the respective different sites are simultaneously bound with the polypeptide to be analyzed, or, one of the two antibodies that recognize the respective different sites is bound with the polypeptide to be analyzed, and then, the other one of the two antibodies is bound with the polypeptide to be analyzed. That is, it is necessary that the two antibodies are simultaneously bound with the two different sites (epitopes) of the polypeptide to be analyzed, and thereby the polypeptide to be analyzed is in a sandwiched state with the two antibodies

Even though the two different recognizing sites (epitopes) of the polypeptide that are to be bound respectively by the two antibodies are distant from each other as a primary structure of the polypeptide, there is the case where the two epitopes are near to each other in a space distance due to polypeptide conformation. In case where the two epitopes of the polypeptide to be analyzed are near to each other in a space distance, it is difficult that the two antibodies are simultaneously bound with the two epitopes. This causes a decrease in sensitivity of a sandwich immunoassay method. For example, in a sandwich immunoassay method for a peptide fragment Aβ, the peptide fragment is sandwiched with an antibody that recognizes the N-terminal of the peptide fragment and an antibody that recognizes the C-terminal of the peptide fragment. In this case, although the two different recognizing sites (epitopes) by the two antibodies are distant from each other as a primary structure of the polypeptide, the two epitopes are near to each other in a space distance (Non-Patent Document 4) . For this reason, there is a possibility it causes a decrease in sensitivity of a sandwich immunoassay method.

An object of the present invention is to provide a sandwich immunoassay method that is capable of detecting and quantifying a polypeptide to be analyzed with a high sensitivity. In particular, an object of the present invention is to provide a sandwich immunoassay method that is capable of detecting and quantifying an Aβ and an Aβ related peptide to be analyzed with a high sensitivity. Furthermore, an object of the present invention is to provide a kit for sandwich immunoassay method for a polypeptide.

### MEANS FOR SOLVING THE PROBLEMS

As a result of diligent efforts, the present inventor, in order to produce an antibody that recognizes an N-terminal of an APP669-x, has immunized a KLH (keyhole limpet hemocyanin) conjugated synthetic peptide VKMC to mice, and conducted cell fusion and screening, and thereby has obtained a hybridoma that produce an antibody that recognizes the N-terminal of the APP669-x. The present inventor has constructed an immunoassay method, such as a sandwich ELISA, that specifically quantify APP669-x using said antibody. Hereby, the present inventor has also constructed an ELISA kit for APP669-x.

The present invention includes the following aspects.
(1) An APP669-x N-terminal-recognizing monoclonal antibody that specifically recognizes an N-terminal of an APP669-x peptide.
(2) An immunoassay method that uses an APP669-x N-terminal-recognizing monoclonal antibody that specifically recognizes an N-terminal of an APP669-x peptide, and comprises reacting an APP669-x in a sample with the APP669-x N-terminal-recognizing monoclonal antibody to measure the APP669-x.
(3) The immunoassay method according to the above item (2), wherein the method is selected from the group consisting of a sandwich immunoassay method, a direct ELISA, an indirect ELISA, a competitive ELISA, a western blotting, an immunohistochemistry, a flow cytometry, an immunoprecipitation, an affinity chromatography, and an immunocytochemistry.
(4) The immunoassay method according to the above item (3), wherein the method uses, in the sandwich immunoassay method,
   the APP669-x N-terminal-recognizing monoclonal antibody, as a first antibody; and
   an anti-Aβ antibody, as a second antibody, capable of recognizing a portion of the APP669-x that is different from the antigen binding site of the first antibody, or a C-terminal of a fragment of the APP669-x.
(5) The immunoassay method according to the above item (3) or (4), wherein the second antibody is a monoclonal antibody or a polyclonal antibody that is capable of recognizing a C-terminal of APP669-711.
(6) The immunoassay method according to any one of the above items (3) to (5), wherein the sandwich immunoassay method is selected from the group consisting of an enzyme immunoassay method (EIA), a radio immunoassay method (RIA), a chemiluminescent immunoassay method (CIA), a fluorescent immunoassay method (FIA), an electro-chemiluminescent immunoassay method (ECLIA), a bioluminescent immunoassay method (BLIA), an immuno-PCR, a turbidimetric immunoassay method (TAI), and a latex agglutination turbidimetric method (LA) .
(7) The immunoassay method according to any one of the above items (3) to (6), wherein the immunoassay method comprises adding an antigen affinity substance capable of binding to the APP669-x to the sample.
(8) The immunoassay method according to the above item (7), wherein the antigen affinity substance is added to the sample, and then, the APP669-x peptide is reacted with the first antibody and the second antibody; or
   while the APP669-x peptide is reacted with the first antibody and the second antibody, the antigen affinity substance is added to a reaction system; or
   the APP669-x peptide is reacted with either one of the first antibody and the second antibody, and then, the antigen affinity substance is added to a reaction system, and then, the APP669-x peptide is reacted with the other one of the first antibody and the second antibody.
(9) The immunoassay method according to the above item (7) or (8), wherein the antigen affinity substance is selected from the group consisting of an antibody, a peptide, a low molecule compound, and a nucleic acid aptamer.
(10) The immunoassay method according to any one of the above items (2) to (9), wherein the sample is a living body-derived sample selected from the group consisting of blood, cerebrospinal fluid, urine, feces, and body secreting fluid.
(11) A kit for sandwich immunoassay method for APP669-711, comprising:
   an APP669-x N-terminal-recognizing monoclonal antibody, as a first antibody, that specifically recognizes an N-terminal of an APP669-x peptide; and
   a monoclonal antibody or a polyclonal antibody, as a second antibody, that is capable of recognizing a C-terminal of APP669-711.
(12) A kit for sandwich immunoassay method for APP669-711 according to the above item (11), further comprising an antigen affinity substance capable of binding to the APP669-x.

Furthermore, as a result of diligent efforts, the present inventor has found that a sandwich immunoassay method is obtained that is capable of detecting and quantifying a polypeptide to be analyzed with a high sensitivity, by changing conformation of the polypeptide to be analyzed and performing a treatment for keeping two epitopes of the polypeptide away from each other in a space distance, wherein the two epitopes are to be bound respectively by the two antibodies used in the sandwich immunoassay method; and specifically by binding a substance which has an affinity to the polypeptide to be analyzed (namely, an antigen affinity substance) to said polypeptide to be analyzed, thereby changing the conformation of said polypeptide to be analyzed.

In the present description, the polypeptide includes a peptide and a protein. The protein includes also a protein after a post translational protein modification, such as a glycoprotein and a phosphorylated protein.

In the present description, "Aβ" is used as an abbreviation of an amyloid β peptide. That is, "Aβ" includes Aβ1-40 and Aβ1-42. A Peptide other than the Aβ generated by cleavage of amyloid precursor protein (APP) may be referred to as an Aβ related peptide (or, an Aβ-like peptide). Aβ and an Aβ related peptide (or, an Aβ-like peptide) that are generated by cleavage of amyloid precursor protein (APP) may be referred to as "APP-derived peptide".

The present invention includes the following aspects.
(B-1) A sandwich immunoassay method for a polypeptide, which uses a first antibody having an antigen binding site capable of recognizing a target polypeptide in a sample, and a second antibody having an antigen binding site that is different from the antigen binding site of the first antibody and is capable of recognizing the target polypeptide,
   wherein the immunoassay method comprises adding an antigen affinity substance capable of binding to the target polypeptide to the sample.
(B-2) The immunoassay method according to the above item (B-1), wherein the antigen affinity substance is added to the sample containing the target polypeptide, and then, the target polypeptide is reacted with the first antibody and the second antibody; or
   while the target polypeptide is reacted with the first antibody and the second antibody, the antigen affinity substance is added to a reaction system; or
   the target polypeptide is reacted with either one of the first antibody and the second antibody, and then, the antigen affinity substance is added to a reaction system, and then, the target polypeptide is reacted with the other one of the first antibody and the second antibody.
(B-3) The immunoassay method according to the above item (B-1) or (B-2), wherein the antigen affinity substance is selected from the group consisting of an antibody, a peptide, a low molecule compound, and a nucleic acid aptamer.
(B-4) The immunoassay method according to any one of the above items (B-1) to (B-3), wherein sandwich immunoassay method is selected from the group consisting of an enzyme immunoassay method (EIA), a radio immunoassay method (RIA), a chemiluminescent immunoassay method (CIA), a fluorescent immunoassay method (FIA), an electro-chemiluminescent immunoassay method (ECLIA), a bioluminescent immunoassay method (BLIA), an immuno-PCR, a turbidimetric immunoassay method (TAI), and a latex agglutination turbidimetric method (LA) .
(B-5) The immunoassay method according to any one of the above items (B-1) to (B-4), wherein the first antibody is an antibody that recognizes an N-terminal of the target polypeptide, and the second antibody is an antibody that recognizes a C-terminal of the target polypeptide.
(B-6) The immunoassay method according to the above item (B-5), wherein the antigen affinity substance is a substance that does not act on a neighborhood of the N-terminal of the target polypeptide, and does not act on a neighborhood of the C-terminal of the target polypeptide.
(B-7) The immunoassay method according to the above item (B-5) or (B-6), wherein the antigen affinity substance is a substance that act on an intermediate portion comprising a portion from a fourth residue site from the N-terminal of the target polypeptide, to a fourth residue site from the C-terminal of the target polypeptide.
(B-8) The immunoassay method according to any one of the above items (B-1) to (B-7), wherein the target polypeptide is selected from the group consisting of an Aβ and an Aβ related peptide.
(B-9) The immunoassay method according to any one of the above items (B-1) to (B-8), wherein the sample is a living body-derived sample selected from the group consisting of blood, cerebrospinal fluid, urine, feces, and body secreting fluid.
(B-10) A kit for sandwich immunoassay method for a polypeptide, comprising:
   a first antibody having an antigen binding site capable of recognizing a target polypeptide;
   a second antibody having an antigen binding site that is different from the antigen binding site of the first antibody and is capable of recognizing the target polypeptide; and
   an antigen affinity substance capable of binding to the target polypeptide.

### EFFECTS OF THE INVENTION

The present invention provides an antibody that specifically recognizes an N-terminal of an APP669-x. The present invention provides an immunoassay method for APP669-x, such as a sandwich immunoassay method, an ELISA, a western blotting, an immunohistochemistry, a flow cytometry, an immunoprecipitation, an affinity chromatography, and an immunocytochemistry, using said antibody that specifically recognizes an N-terminal of an APP669-x.

The present invention provides, as a preferred embodiment, a sandwich ELISA method for APP669-711. The present invention also provides a reagent kit for conducting a sandwich ELISA method for APP669-711. Although a mass spectrometry (MALDI-TOF MS) is useful as an analysis technique for biomarkers such as Aβ related peptides including APP669-711, a sandwich ELISA is a widely and generally used measurement method as a clinical examination method and is low cost method. Accordingly, the sandwich ELISA can be a more useful analysis technique.

In a sandwich immunoassay method of the present invention, an antigen affinity substance capable of binding to a target polypeptide is added to a sample. By this, conformation of the target polypeptide is changed, and two epitopes of the polypeptide is kept away from each other in a space distance, wherein the two epitopes are to be bound respectively by the two antibodies used in the sandwich immunoassay method. Accordingly, antigen-antibody reactions at the two sites proceed successfully, the target polypeptide can be detected and quantified with a high sensitivity.

Specific Aβ polypeptides (Aβ1-40, and Aβ1-42) and Aβ related peptides (for example, Aβ1-39, APP669-711) exist in a living body, and these peptides have been attracting attention as a biomarker for the Alzheimer's disease. As for these Aβs and Aβ related peptides, in particular, as for Aβ related peptides, a sandwich immunoassay method capable of detecting and quantifying the peptides with a high sensitivity is desired. The present invention provides, as for these Aβs and Aβ related peptides, in particular, as for Aβ related peptides, a sandwich immunoassay method capable of detecting and quantifying the peptides with a high sensitivity.

### BRIEF DESCRIPTION OF DRAWINGS

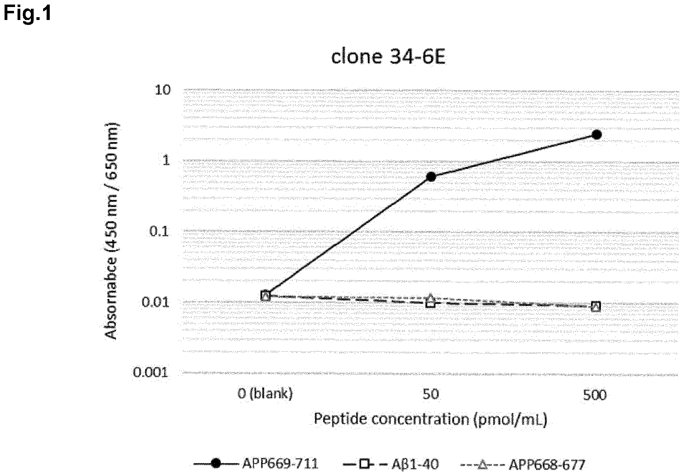

[Fig.1] Fig. 1 is a graph showing results of a direct ELISA using clone 34-6E of the antibody specific to the N-terminal of APP669-x, in Example 1-2. The horizontal axis indicates peptide concentration (pmol/mL), and the vertical axis indicates absorbance of main wavelength 450nm / sub wavelength 650nm measured by a microplate reader.

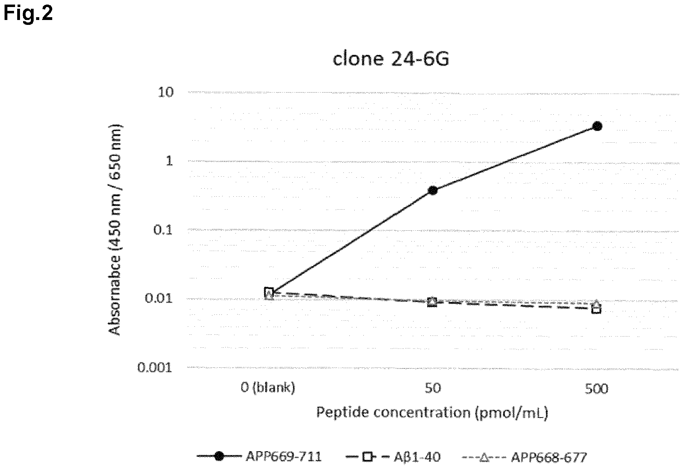

[Fig.2] Fig. 2 is a graph showing results of a direct ELISA using clone 24-6G of the antibody specific to the N-terminal of APP669-x, in Example 1-2. The horizontal axis indicates peptide concentration (pmol/mL), and the vertical axis indicates absorbance of main wavelength 450nm / sub wavelength 650nm measured by the microplate reader.

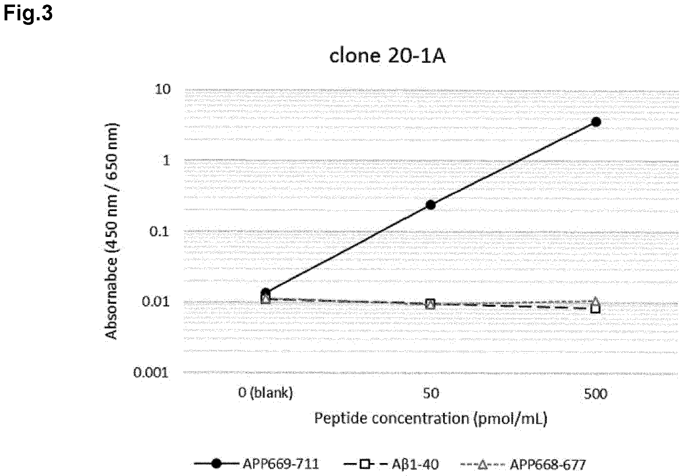

[Fig.3] Fig. 3 is a graph showing results of a direct ELISA using clone 20-1A of the antibody specific to the N-terminal of APP669-x, in Example 1-2. The horizontal axis indicates peptide concentration (pmol/mL), and the vertical axis indicates absorbance of main wavelength 450nm / sub wavelength 650nm measured by the microplate reader.

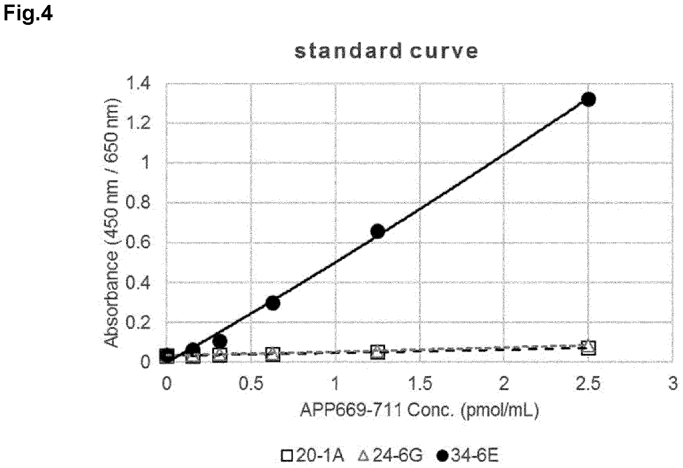

[Fig.4] Fig. 4 is a graph showing results of a sandwich ELISA for APP669-711 using three clones of the antibodies specific to the N-terminal of APP669-x, respectively, in Example 2-1. The horizontal axis indicates APP669-711 concentration (pmol/mL), and the vertical axis indicates absorbance of main wavelength 450nm / sub wavelength 650nm measured by the microplate reader.

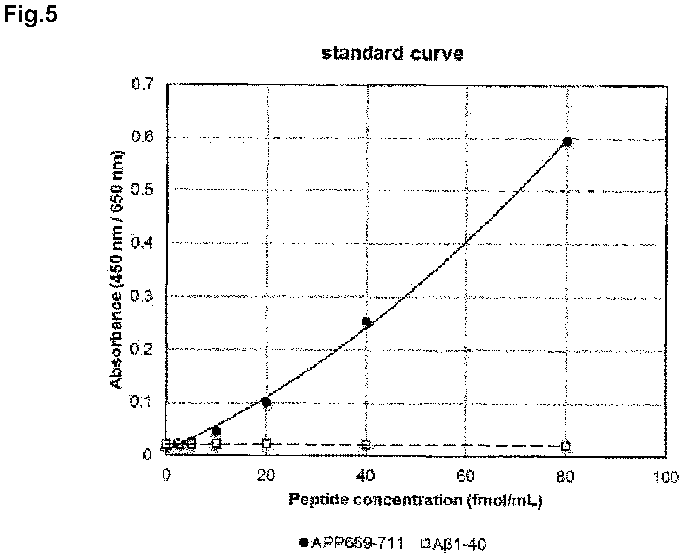

[Fig.5] Fig. 5 is a graph showing specificity of a sandwich ELISA for APP669-711 in case using clone 34-6E of the antibody specific to the N-terminal of APP669-x, in Example 2-2. The horizontal axis indicates peptide concentration (pmol/mL), and the vertical axis indicates absorbance of main wavelength 450nm / sub wavelength 650nm measured by the microplate reader.

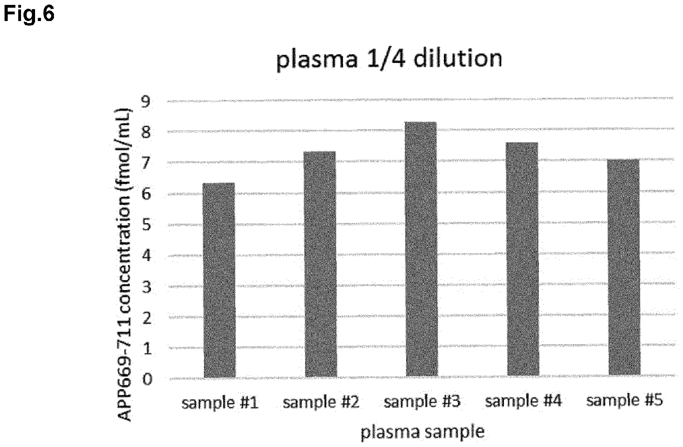

[Fig.6] Fig. 6 is a graph showing results of measuring a human plasma by a sandwich ELISA for APP669-711, in Example 2-4. The horizontal axis indicates each plasma sample, and the vertical axis indicates APP669-711 concentration (fmol/mL).

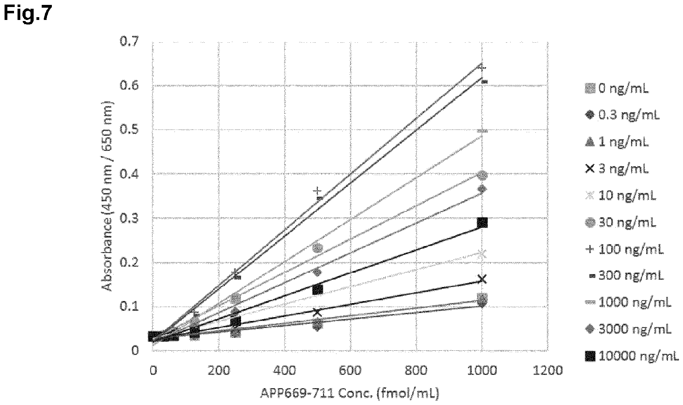

[Fig.7] Fig. 7 is a graph showing calibration curves of ELISA for APP669-711 by adding anti-Aβ antibody 4G8, in Example B1. The horizontal axis indicates APP669-711 concentration (fmol/mL), and the vertical axis indicates absorbance of 450nm/650nm. The calibration curves are represented with respect to each concentration of anti-Aβ antibody 4G8.

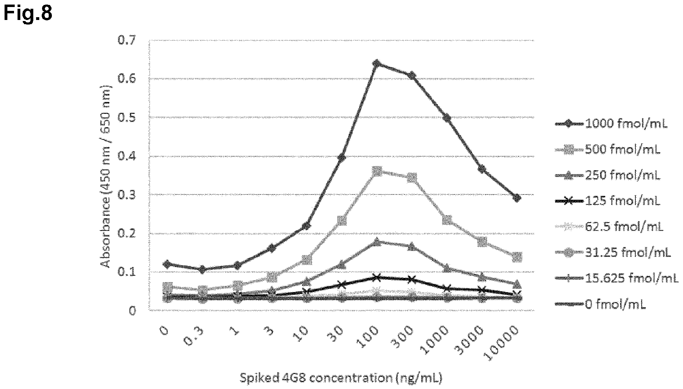

[Fig.8] Fig. 8 is a graph showing reactivity of ELISA for APP669-711 by adding anti-Aβ antibody 4G8, in Example B1. The horizontal axis indicates concentration (ng/mL) of anti-Aβ antibody 4G8, and the vertical axis indicates absorbance of 450nm/650nm. The points are connected by a line with respect to each concentration of APP669-711.

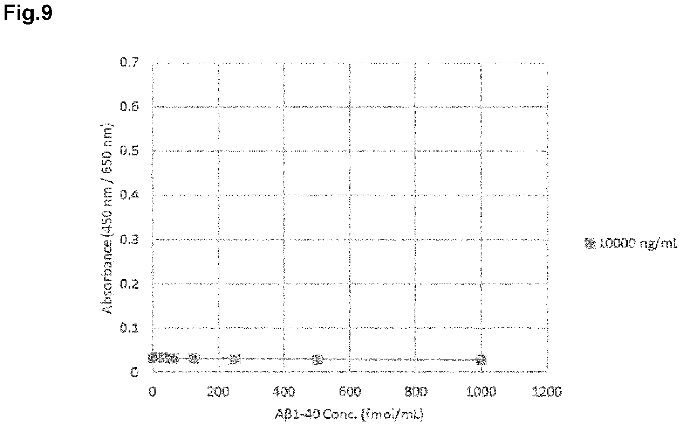

[Fig.9] Fig. 9 is a graph showing reactivity of ELISA for APP669-711 against Aβ1-40 by adding anti-Aβ antibody 4G8, in Example B1. The horizontal axis indicates concentration (fmol/mL) of Aβ1-40, and the vertical axis indicates absorbance of 450nm/650nm.

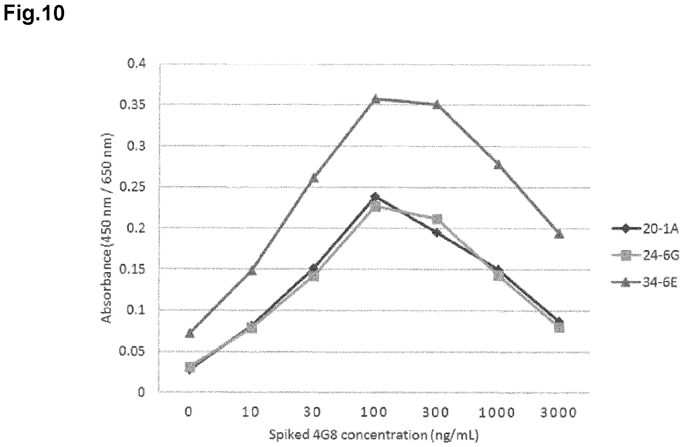

[Fig.10] Fig. 10 is a graph showing adding effect of anti-Aβ antibody 4G8 in ELISA for APP669-711 using three clones of the antibodies recognizing the N-terminal, respectively, in Example B2. The horizontal axis indicates concentration (ng/mL) of anti-Aβ antibody 4G8, and the vertical axis indicates absorbance of 450nm/650nm. The points are connected by a line with respect to each clone of the antibody recognizing the N-terminal.

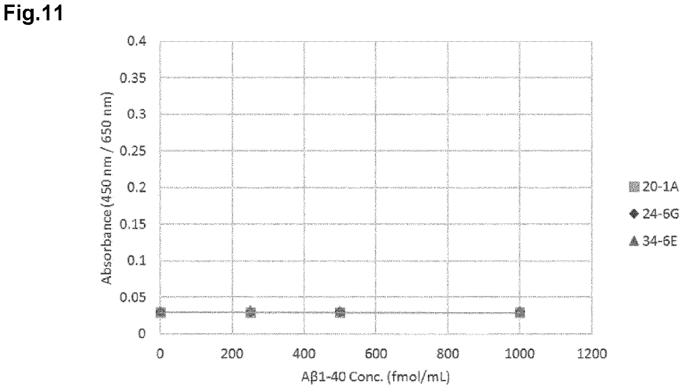

[Fig.11] Fig. 11 is a graph showing reactivity of ELISA for APP669-711 against Aβ1-40 by adding anti-Aβ antibody 4G8, in Example B2. The three clones of the antibodies recognizing the N-terminal were used, respectively. The horizontal axis indicates concentration (fmol/mL) of Aβ1-40, and the vertical axis indicates absorbance of 450nm/650nm.

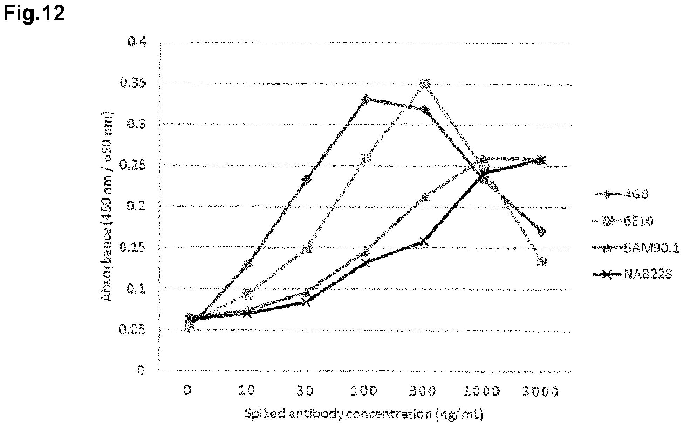

[Fig.12] Fig. 12 is a graph showing adding effect of four clones of anti-Aβ antibodies in ELISA for APP669-711, respectively, in Example B3. The horizontal axis indicates concentration (ng/mL) of anti-Aβ antibodies, and the vertical axis indicates absorbance of 450nm/650nm. The points are connected by a line with respect to each clone of the anti-Aβ antibody.

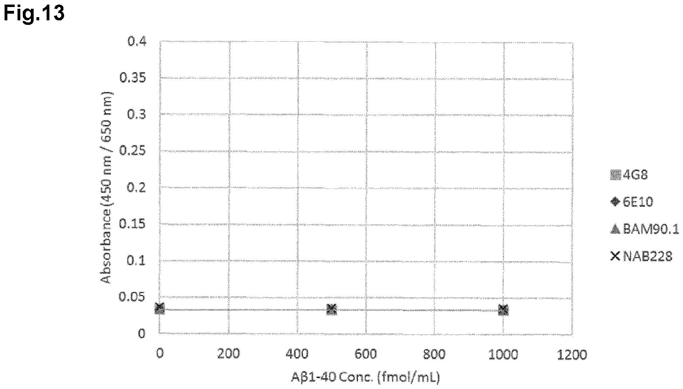

[Fig.13] Fig. 13 is a graph showing reactivity of ELISA for APP669-711 against Aβ1-40 by adding anti-Aβ antibody, in Example B3. The four clones of the anti-Aβ antibodies were used, respectively. The horizontal axis indicates concentration (fmol/mL) of Aβ1-40, and the vertical axis indicates absorbance of 450nm/650nm.

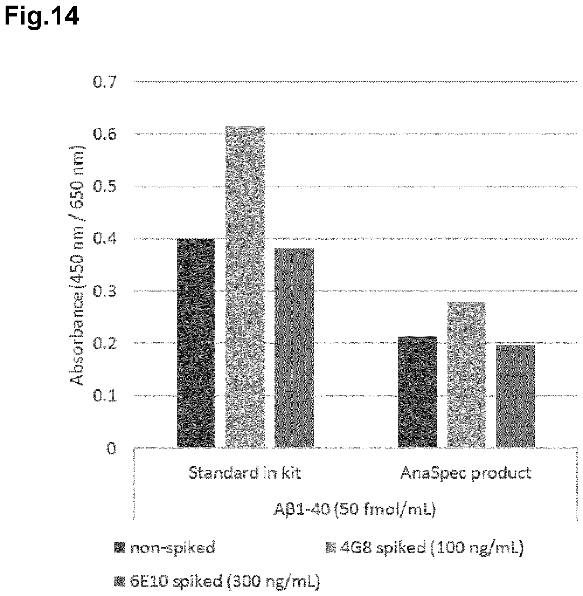

[Fig.14] Fig. 14 is a graph showing adding effect of anti-Aβ antibodies 4G8 and 6E10, respectively, in ELISA for APP669-711, in Example B4. The vertical axis indicates absorbance of 450nm/650nm. As the samples, the standard contained in the ELISA kit, and Aβ1-40 available from AnaSpec were used, respectively.

### MODES FOR CARRYING OUT THE INVENTION

### [1. APP669-x N-terminal-recognizing monoclonal antibody]

An APP669-x N-terminal-recognizing monoclonal antibody of the present invention is an antibody that specifically recognizes an N-terminal of an APP669-x peptide.

The present inventor, in order to produce an antibody that recognizes an N-terminal of an APP669-x, has immunized a KLH conjugated synthetic peptide VKMC (SEQ ID NO: 5) to mice, and conducted cell fusion and screening, and thereby has obtained a hybridoma that produce an antibody that recognizes the N-terminal of the APP669-x. More detailed production of the antibody is described in the Examples.

This antibody recognizes the N-terminal of the APP669-x, accordingly, the antibody can specifically recognize the N-terminal of the various peptides belonging to the APP669-x, that includes APP669-711 (SEQ ID NO: 3). Therefore, by using the APP669-x N-terminal-recognizing monoclonal antibody, an immunoreaction between the APP669-x in a sample and the APP669-x N-terminal-recognizing monoclonal antibody can occur.

### [2. Target APP669-x peptide]

An amyloid precursor protein (APP) is a single-pass transmembrane protein and is composed of 770 amino acid residues . The amyloid precursor protein (APP) is subjected to proteolysis by β secretase and γ secretase, and an amyloid beta peptide (Aβ) is produced by the proteolysis. APP672-711 and Aβ1-40 indicate the same peptide (SEQ ID NO.: 1). APP672-713 and Aβ1-42 indicate the same peptide (SEQ ID NO. : 2). APP669-711 (SEQ ID NO: 3) belongs to the APP669-x. Here, "x" is higher than 669, and for example, higher than 677 regarding the lower limit; and is not particularly limited, and is 770, or smaller than 770 regarding the upper limit. However, the upper limit of "x" is not particularly limited, considering the spirit of the present invention, that is, the APP669-x N-terminal-recognizing monoclonal antibody can specifically recognize the N-terminal of the APP669-x peptide, and the immunoreaction between them can occur. Examples of the APP669-x peptides include APP669-711 (SEQ ID NO: 3), APP669-709, APP669-710, APP669-713, and the like.
APP672-711 (Aβ1-40) (SEQ ID NO: 1):
   DAEFRHDSGYEVHHQKLVFFAEDVGSNKGAIIGLMVGGVV
APP672-713 (Aβ1-42) (SEQ ID NO: 2):
   DAEFRHDSGYEVHHQKLVFFAEDVGSNKGAIIGLMVGGVVIA
APP669-711 (SEQ ID NO: 3):
   VKMDAEFRHDSGYEVHHQKLVFFAEDVGSNKGAIIGLMVGGVV

### [3. Immunoassay method]

An immunoassay method of the present invention uses an APP669-x N-terminal-recognizing monoclonal antibody that specifically recognizes an N-terminal of an APP669-x peptide, and comprises reacting an APP669-x in a sample with the APP669-x N-terminal-recognizing monoclonal antibody to measure the APP669-x.

As to the immunoassay method, examples of the various immunoassay methods include a sandwich immunoassay method, a direct ELISA, an indirect ELISA, a competitive ELISA, a western blotting, an immunohistochemistry, a flow cytometry, an immunoprecipitation, an affinity chromatography, an immunocytochemistry, and the like. According to the ordinary procedure, the immunoassay method can be performed by using the APP669-x N-terminal-recognizing monoclonal antibody of the present invention.

In the sandwich immunoassay method, said method can use, the APP669-x N-terminal-recognizing monoclonal antibody, as a first antibody; and an anti-Aβ antibody, as a second antibody, capable of recognizing a portion of the APP669-x that is different from the antigen binding site of the first antibody, or a C-terminal of a fragment of the APP669-x.

As the second antibody, it is advisable to use an anti-Aβ antibody that is capable of recognizing a C-terminal of the APP669-x fragment. In case that the target peptide to be measured is APP669-711, a monoclonal antibody that is capable of recognizing the C-terminal of APP669-711 is used. The C-terminal of Aβ1-40 is the same as the C-terminal of APP669-711, and an antibody that is capable of recognizing such C-terminal is commercially available.

Examples of the sandwich immunoassay methods include an enzyme immunoassay method (EIA), a radio immunoassay method (RIA), a chemiluminescent immunoassay method (CIA), a fluorescent immunoassay method (FIA), an electro-chemiluminescent immunoassay method (ECLIA), a bioluminescent immunoassay method (BLIA), an immuno-PCR, a turbidimetric immunoassay method (TAI), a latex agglutination turbidimetric method (LA), and the like.

In the sandwich immunoassay method, according to the ordinary procedure, it is advisable to perform an immunoreaction by adding the first antibody and the second antibody consecutively or simultaneously to a sample containing the APP669-x peptide.

Furthermore, in the present invention, an antigen affinity substance capable of binding to the APP669-x may be added to the sample containing the APP669-x peptide.

In the above case, it is possible that the antigen affinity substance is added to the sample, and then, the APP669-x peptide is reacted with the first antibody and the second antibody; or
while the APP669-x peptide is reacted with the first antibody and the second antibody, the antigen affinity substance is added to a reaction system; or
the APP669-x peptide is reacted with either one of the first antibody and the second antibody, and then, the antigen affinity substance is added to a reaction system, and then, the APP669-x peptide is reacted with the other one of the first antibody and the second antibody.

By adding the antigen affinity substance capable of binding to the target polypeptide into the sample, conformation of the target polypeptide is changed, and two epitopes of the polypeptide is kept away from each other in a space distance, wherein the two epitopes are to be bound respectively by the two antibodies used in the sandwich immunoassay method. Accordingly, antigen-antibody reactions at the two sites proceed successfully, the target polypeptide can be detected and quantified with a high sensitivity.

Regarding the antigen affinity substance, a substance having an affinity to the target polypeptide and capable of binding to the target polypeptide can serves as the antigen affinity substance. Examples of the antigen affinity substances include an antibody, a peptide, a low molecule compound, a nucleic acid aptamer, and the like. Here, the "binding" includes a binding by an intermolecular interaction such as an electrostatic interaction, a Van der Waals force, a hydrogen bonding, a hydrophobic interaction, a dipole interaction, a dispersion force, and the like. Any antigen affinity substance may be used, so long as said substance can change the conformation of the target polypeptide, and keep two epitopes of the polypeptide away from each other in a space distance, wherein the two epitopes are to be bound respectively by the two antibodies used in the sandwich immunoassay method.

As the antibody included in the antigen affinity substance, it is advisable to use an antibody having an antigen binding site that is different from the antigen binding sites of the first antibody and the second antibody.

As the peptide included in the antigen affinity substance, it is advisable to use a peptide composed of 2 to 12 amino acid residues. For example, as a peptide which binds to Aβ, iAβ5 (5 amino acids), D3 (12 amino acids), NH2-D-Trp-Aib-OH (2 amino acids), and so on are mentioned.

As the low molecule compound included in the antigen affinity substance, it is advisable to use a low molecule compound capable of binding to the target polypeptide. For example, a low molecule compound which binds to a certain protein, such as used in the drug discovery is mentioned. As a low molecule compound which binds to Aβ, Scyllo-inositol and so on are mentioned. Various inhibitors (for example, various Stemolecule ™ low molecule compounds) are also mentioned.

As the nucleic acid aptamer included in the antigen affinity substance, a DNA aptamer, an RNA aptamer, and so on are mentioned.

An addition amount of the antigen affinity substance depends on a kind or an amount of the target polypeptide in the sample, and a kind of the antigen affinity substance. The addition amount of the antigen affinity substance is not particularly limited, and may be an amount such that said antigen affinity substance binds to the target polypeptide to change the conformation of said target polypeptide. The addition amount of the antigen affinity substance may be, as expressed in a concentration of the antigen affinity substance in the sample containing the target polypeptide, for example, about 0.1 ng/mL to 100,000 ng/mL, preferably about 0.1 ng/mL to 10,000 ng/mL, more preferably about 0.1 ng/mL to 3, 000 ng/mL.

As mentioned above, in the sandwich immunoassay method, it is possible that the method use the APP669-x N-terminal-recognizing monoclonal antibody, as a first antibody; and an anti-Aβ antibody, as a second antibody, capable of recognizing a portion of the APP669-x that is different from the antigen binding site of the first antibody, or a C-terminal of a fragment of the APP669-x.

In the above case, the antigen affinity substance is preferably a substance that does not act on a neighborhood of the N-terminal of the target polypeptide, and does not act on a neighborhood of the C-terminal of the target polypeptide. It is not preferred that a competition or an inhibition to the target polypeptide occurs between the antigen affinity substance and the first antibody, and also that a competition or an inhibition to the target polypeptide occurs between the antigen affinity substance and the second antibody.

More specifically, the antigen affinity substance is preferably a substance that act on an intermediate portion comprising a portion from a fourth residue site from the N-terminal of the target polypeptide, to a fourth residue site from the C-terminal of the target polypeptide. The antigen affinity substance is more preferably a substance that act on an intermediate portion comprising a portion from a sixth residue site from the N-terminal of the target polypeptide, to a sixth residue site from the C-terminal of the target polypeptide.

In the case of a sandwich ELISA (Enzyme-Linked ImmunoSorbent Assay) utilizing an enzyme as a labeling substance, it is advisable to use an N-terminal-recognizing antibody of the target polypeptide, as the first antibody, is used as an immobilized antibody (a capture antibody); and to use a C-terminal-recognizing antibody of the target polypeptide, as the second antibody, is used as a detection antibody (a labeled antibody).

The sandwich immunoassay method of the present invention is applicable to the sandwich ELISA utilizing an enzyme as a labeling substance, and furthermore, a radio immunoassay method (RIA) utilizing a radioisotope, a chemiluminescent immunoassay method (CIA) utilizing a chemiluminescent substance, a fluorescent immunoassay method (FIA) utilizing a fluorescent substance, an electro-chemiluminescent immunoassay method (ECLIA) utilizing a metal complex, a bioluminescent immunoassay method (BLIA) utilizing a bioluminescent substance such as a luciferase, an immuno-PCR comprising amplifying a nucleic acid that labeled an antibody by PCR and detecting the nucleic acid, a turbidimetric immunoassay method (TAI) detecting turbidity occurred by forming an immunocomplex, a latex agglutination turbidimetric method (LA) detecting a latex aggregated by forming an immunocomplex, an immunochromatography assay utilizing a reaction on a cellulose membrane, and the like.

In the present invention, a basic operation of the sandwich immunoassay method, even if said method includes adding the antigen affinity substance to the sample, can be conducted according to the known operation.

In the present invention, the target APP669-x peptide is contained in a living body sample. The living body sample includes body fluids such as blood, cerebrospinal fluid (CSF), urine, body secreting fluid, saliva, sputum and the like; and feces. The blood sample includes whole blood, plasma, serum and the like. The blood sample can be prepared by appropriately treating whole blood collected from an individual. The treatment performed in the case of preparing a blood sample from collected whole blood is not particularly limited, and any treatment that is clinically acceptable may be performed. For example, centrifugal separation or the like may be performed. The blood sample may be appropriately stored at a low temperature such as freezing in the intermediate stage of the preparation step or in the post stage of the preparation step. In the present invention, the living body sample is disposed of rather than being returned to the individual from which it is derived. The blood sample is preferably used as a target sample to be measured, since collecting the blood sample is minimally invasive than the sample such as solid sample, and cerebrospinal fluid; and the blood sample is a target sample to be measured for screening various diseases in a general medical examination, or a complete physical examination.

A kit for sandwich immunoassay method for APP669-711 of the present invention is for conducting the above mentioned sandwich immunoassay method. The kit comprises:
an APP669-x N-terminal-recognizing monoclonal antibody, as a first antibody, that specifically recognizes an N-terminal of an APP669-x peptide; and
a monoclonal antibody or a polyclonal antibody, as a second antibody, that is capable of recognizing a C-terminal of APP669-711.

The kit may further comprise an antigen affinity substance capable of binding to the APP669-x. Furthermore, the kit can contain various components used for operations in the sandwich immunoassay method, such as a diluting solution for use of a preparation of sample solution, a washing solution, and the like.

An immunoassay method of the present invention is a sandwich immunoassay method for a polypeptide, which uses a first antibody having an antigen binding site capable of recognizing a target polypeptide in a sample, and a second antibody having an antigen binding site that is different from the antigen binding site of the first antibody and is capable of recognizing the target polypeptide,
wherein the immunoassay method comprises adding an antigen affinity substance capable of binding to the target polypeptide to the sample.

In the present invention, it is possible that the antigen affinity substance is added to the sample containing the target polypeptide, and then, the target polypeptide is reacted with the first antibody and the second antibody; or
while the target polypeptide is reacted with the first antibody and the second antibody, the antigen affinity substance is added to a reaction system; or
the target polypeptide is reacted with either one of the first antibody and the second antibody, and then, the antigen affinity substance is added to a reaction system, and then, the target polypeptide is reacted with the other one of the first antibody and the second antibody.

By adding the antigen affinity substance capable of binding to the target polypeptide into the sample, conformation of the target polypeptide is changed, and two epitopes of the polypeptide is kept away from each other in a space distance, wherein the two epitopes are to be bound respectively by the two antibodies used in the sandwich immunoassay method. Accordingly, antigen-antibody reactions at the two sites proceed successfully, the target polypeptide can be detected and quantified with a high sensitivity.

Regarding the antigen affinity substance, a substance having an affinity to the target polypeptide and capable of binding to the target polypeptide can serves as the antigen affinity substance. Examples of the antigen affinity substances include an antibody, a peptide, a low molecule compound, a nucleic acid aptamer, and the like. Here, the "binding" includes a binding by an intermolecular interaction such as an electrostatic interaction, a Van der Waals force, a hydrogen bonding, a hydrophobic interaction, a dipole interaction, a dispersion force, and the like. Any antigen affinity substance may be used, so long as said substance can change the conformation of the target polypeptide, and keep two epitopes of the polypeptide away from each other in a space distance, wherein the two epitopes are to be bound respectively by the two antibodies used in the sandwich immunoassay method.

As the antibody included in the antigen affinity substance, it is advisable to use an antibody having an antigen binding site that is different from the antigen binding sites of the first antibody and the second antibody.

As the peptide included in the antigen affinity substance, it is advisable to use a peptide composed of 2 to 12 amino acid residues. For example, as a peptide which binds to Aβ, iAβ5 (5 amino acids), D3 (12 amino acids), NH2-D-Trp-Aib-OH (2 amino acids), and so on are mentioned.

As the low molecule compound included in the antigen affinity substance, it is advisable to use a low molecule compound capable of binding to the target polypeptide. For example, a low molecule compound which binds to a certain protein, such as used in the drug discovery is mentioned. As a low molecule compound which binds to Aβ, Scyllo-inositol and so on are mentioned. Various inhibitors (for example, various Stemolecule ™ low molecule compounds) are also mentioned.

As the nucleic acid aptamer included in the antigen affinity substance, a DNA aptamer, an RNA aptamer, and so on are mentioned.

An addition amount of the antigen affinity substance depends on a kind or an amount of the target polypeptide in the sample, and a kind of the antigen affinity substance. The addition amount of the antigen affinity substance is not particularly limited, and may be an amount such that said antigen affinity substance binds to the target polypeptide to change the conformation of said target polypeptide. The addition amount of the antigen affinity substance may be, as expressed in a concentration of the antigen affinity substance in the sample containing the target polypeptide, for example, about 0.1 ng/mL to 100,000 ng/mL, preferably about 0.1 ng/mL to 10,000 ng/mL, more preferably about 0.1 ng/mL to 3,000 ng/mL.

In the present invention, various antibodies used in the sandwich immunoassay method can be used, as the first antibody having an antigen binding site capable of recognizing a target polypeptide in a sample, and the second antibody having an antigen binding site that is different from the antigen binding site of the first antibody and is capable of recognizing the target polypeptide.

For example, an antibody that recognizes an N-terminal of the target polypeptide can be used as the first antibody, and an antibody that recognizes a C-terminal of the target polypeptide can be used as the second antibody.

In the above case, the antigen affinity substance is preferably a substance that does not act on a neighborhood of the N-terminal of the target polypeptide, and does not act on a neighborhood of the C-terminal of the target polypeptide. It is not preferred that a competition or an inhibition to the target polypeptide occurs between the antigen affinity substance and the first antibody, and also that a competition or an inhibition to the target polypeptide occurs between the antigen affinity substance and the second antibody.

More specifically, the antigen affinity substance is preferably a substance that act on an intermediate portion comprising a portion from a fourth residue site from the N-terminal of the target polypeptide, to a fourth residue site from the C-terminal of the target polypeptide. The antigen affinity substance is more preferably a substance that act on an intermediate portion comprising a portion from a sixth residue site from the N-terminal of the target polypeptide, to a sixth residue site from the C-terminal of the target polypeptide.

In the case of a sandwich ELISA (Enzyme-Linked ImmunoSorbent Assay) utilizing an enzyme as a labeling substance, it is advisable to use an N-terminal-recognizing antibody of the target polypeptide, as the first antibody, is used as an immobilized antibody (a capture antibody); and to use a C-terminal-recognizing antibody of the target polypeptide, as the second antibody, is used as a detection antibody (a labeled antibody).

The sandwich immunoassay method of the present invention is applicable to the sandwich ELISA utilizing an enzyme as a labeling substance, and furthermore, a radio immunoassay method (RIA) utilizing a radioisotope, a chemiluminescent immunoassay method (CIA) utilizing a chemiluminescent substance, a fluorescent immunoassay method (FIA) utilizing a fluorescent substance, an electro-chemiluminescent immunoassay method (ECLIA) utilizing a metal complex, a bioluminescent immunoassay method (BLIA) utilizing a bioluminescent substance such as a luciferase, an immuno-PCR comprising amplifying a nucleic acid that labeled an antibody by PCR and detecting the nucleic acid, a turbidimetric immunoassay method (TAI) detecting turbidity occurred by forming an immunocomplex, a latex agglutination turbidimetric method (LA) detecting a latex aggregated by forming an immunocomplex, an immunochromatography assay utilizing a reaction on a cellulose membrane, and the like.

In the present invention, a basic operation of the sandwich immunoassay method can be conducted according to the known operation, other than adding step of the antigen affinity substance to the sample.

The present invention is preferably used, in particular, in the case where the target polypeptide is an Aβ and an Aβ related peptide. The Aβs (Aβ1-40, and Aβ1-42) and the Aβ related peptides (for example, Aβ1-39, APP669-711) exist in a living body, and these peptides is attracting attention as a biomarker for the Alzheimer's disease. As for these Aβs and Aβ related peptides, in particular, as for the Aβ related peptides, a sandwich immunoassay method capable of detecting and quantifying the peptides with a high sensitivity has been desired. The present invention provides, as for these Aβs and Aβ related peptides, in particular, as for the Aβ related peptides, a sandwich immunoassay method capable of detecting and quantifying the peptides with a high sensitivity. Examples of the Aβs and the Aβ related peptides are shown below.
APP672-709 (Aβ1-38) (SEQ ID NO: 6):
   DAEFRHDSGYEVHHQKLVFFAEDVGSNKGAIIGLMVGG
APP674-711 (Aβ3-40) (SEQ ID NO: 7):
   EFRHDSGYEVHHQKLVFFAEDVGSNKGAIIGLMVGGVV
APP672-710 (Aβ1-39) (SEQ ID NO: 8):
   DAEFRHDSGYEVHHQKLVFFAEDVGSNKGAIIGLMVGGV
APP672-711 (Aβ1-40) (SEQ ID NO: 1):
   DAEFRHDSGYEVHHQKLVFFAEDVGSNKGAIIGLMVGGVV
OxAPP672-711 (OxAβ1-40) (SEQ ID NO: 9):
   DAEFRHDSGYEVHHQKLVFFAEDVGSNKGAIIGLMVGGVV (Met 706 is oxidized)
APP672-713 (Aβ1-42) (SEQ ID NO: 2):
   DAEFRHDSGYEVHHQKLVFFAEDVGSNKGAIIGLMVGGVVIA
APP669-711 (SEQ ID NO: 3):
   VKMDAEFRHDSGYEVHHQKLVFFAEDVGSNKGAIIGLMVGGVV

An amyloid precursor protein (APP) is a single-pass transmembrane protein and is composed of 770 amino acid residues . The amyloid precursor protein (APP) is subjected to proteolysis by β secretase and γ secretase, and an amyloid beta peptide (Aβ) is produced by the proteolysis. APP672-713 and Aβ1-42 indicate the same peptide (SEQ ID NO.: 2). And, APP672-711 and Aβ1-40 indicate the same peptide (SEQ ID NO.: 1). In the present invention, the target polypeptide includes various Aβ related peptides, which are not limited to the above shown peptides as the examples. Furthermore, the present invention is applicable to various polypeptides other than the Aβs and the Aβ related peptides.

In the present invention, the target polypeptide is contained in a living body sample. The living body sample includes body fluids such as blood, cerebrospinal fluid (CSF), urine, body secreting fluid, saliva, sputum and the like; and feces. The blood sample includes whole blood, plasma, serum and the like. The blood sample can be prepared by appropriately treating whole blood collected from an individual. The treatment performed in the case of preparing a blood sample from collected whole blood is not particularly limited, and any treatment that is clinically acceptable may be performed. For example, centrifugal separation or the like may be performed. The blood sample may be appropriately stored at a low temperature such as freezing in the intermediate stage of the preparation step or in the post stage of the preparation step. In the present invention, the living body sample is disposed of rather than being returned to the individual from which it is derived. The blood sample is preferably used as a target sample to be measured, since collecting the blood sample is minimally invasive than the sample such as solid sample, and cerebrospinal fluid; and the blood sample is a target sample to be measured for screening various diseases in a general medical examination, or a complete physical examination.

A kit for sandwich immunoassay method for a polypeptide of the present invention is for conducting the above mentioned sandwich immunoassay method. The kit comprises:
a first antibody having an antigen binding site capable of recognizing a target polypeptide;
a second antibody having an antigen binding site that is different from the antigen binding site of the first antibody and is capable of recognizing the target polypeptide; and
an antigen affinity substance capable of binding to the target polypeptide.
Furthermore, the kit can contain various components used for operations in the sandwich immunoassay method, such as a diluting solution for use of a preparation of sample solution, a washing solution, and the like.

### EXAMPLES

Hereinafter, the present invention will be described specifically with reference to examples, but is not limited to these examples. In the following, the amount of a matter indicated by % is based on weight when the matter is solid, and based on volume when the matter is liquid unless otherwise indicated.

### [Example 1: Preparation of APP669-x N-terminal-recognizing antibody]

### [Example 1-1: Preparation of monoclonal antibody]

A C-terminal Cys of a synthetic peptide VKMC (SEQ ID NO: 5) was conjugated to a carrier protein (KLH) by using a divalent reactive reagent.

The obtained KLH conjugated synthetic peptide was emulsified by mixing with FCA (Freund's complete adjuvant) using a syringe, and injected (i.e., immunized) into a muscle of a tail joint of a BALB/c mouse in the amount of 200 µg per one mouse. Further, the antigen was emulsified by mixing with FICA (Freund's incomplete adjuvant), and injected subcutaneously (i.e., additionally immunized) into the mouse in the amount of 50 µg per one mouse, two times at an interval of two weeks, and then, finally immunized into an abdominal cavity of the mouse in the amount of 100 µg per one mouse.

After three days from the final immunity, the spleen was collected, and lymphocytes were separated. And then, the lymphocytes were cryopreserved at -80 °C. Collection of the whole blood from the mouse was conducted, and a serum was separated from the blood. And then, the serum was cryopreserved at -40 °C.

The cell fusion of the obtained lymphocytes and mouse myeloma was conducted in the presence of 50 % of polyethylene glycol. The fused cells were dispensed onto eight 96 well microplates to be cultured. After eight days from the cell fusion, the culture supernatant was sampled from the each well of the 96 well microplates, and a primary screening and a secondary screening were conducted sequentially by ELISA. The cell of the positive well was cloned by limiting dilution analysis. Thereafter, a primary screening and a secondary screening were conducted sequentially by ELISA, a hybridoma of the positive clone was cryopreserved.

Thus obtained each hybridoma (clone 20-1A, 24-6G or 34-6E) was cultured on a high density serum-free medium. An antibody was purified from the culture supernatant using Protein A.

### [Example 1-2: Confirmation of specificity of monoclonal antibody by direct ELISA]

Specificity of APP669-x N-terminal-recognizing antibody clone 20-1A, 24-6G or 34-6E was evaluated by a direct ELISA. The direct ELISA was performed as follows.

A synthetic peptide APP669-711 (PEPTIDE INSTITUTE, INC), Aβ1-40 (PEPTIDE INSTITUTE, INC), or APP668-677 (SEQ ID NO: 4), (Toray Research Center, Inc.) was diluted to each concentration of 50, or 500 pmol/mL by a sodium carbonate buffer (pH 9.6). The sequence of the each synthetic peptide is shown in Table 1. The each synthetic peptide solution was added to each well of a 96 well microplate in an amount of 50 µL, and incubated at 4 °C for 2 hours to conduct an immobilization. Further, a blank well was prepared. The solution in the plate was removed, and a 4-fold dilution of Block Ace (DS Pharma) was added to each well in an amount of 100 µL, and incubated at 4 °C for 2 hours to conduct a blocking. The solution in the plate was removed, and washing was conducted with 300 µL of PBST (0.05 % Tween20 in PBS). An APP669-x N-terminal-recognizing antibody that was diluted to a concentration of 0.5 µg/mL by a 10-fold dilution of Block Ace was added to each well in an amount of 50 µL, and incubated at 4 °C for 1 hour. The sample solution in the plate was removed, and washing was conducted with 300 µL of PBST. An HRP-labeled anti-mouse IgG antibody (ZYMED) solution that was diluted 4, 000 times by a 10-fold dilution of Block Ace was added to each well in an amount of 50 µL, and incubated at 4 °C for 1 hour. The solution in the plate was removed, and washing was conducted with 300 µL of PBST. An ELISA POD Substrate TMB Kit (Nacalai) was added to each well in an amount of 100 µL, and an incubation in the dark for 30 minutes was conducted to develop a color. 100 µL of 2N sulfuric acid was added to stop the reaction of the color development. The absorbance of main wavelength 450nm / sub wavelength 650nm was measured by a microplate reader.

These results are shown in Figs. 1 to 3. That is, Fig. 1 is a graph showing results of a direct ELISA using clone 34-6E of the antibody specific to the N-terminal of APP669-x. Fig. 2 is a graph showing results of a direct ELISA using clone 24-6G of the antibody specific to the N-terminal of APP669-x. Fig. 3 is a graph showing results of a direct ELISA using clone 20-1A of the antibody specific to the N-terminal of APP669-x. In Figs. 1 to 3, the horizontal axis indicates peptide concentration (pmol/mL), and the vertical axis indicates absorbance of main wavelength 450nm / sub wavelength 650nm measured by the microplate reader.

From Figs. 1 to 3, it was confirmed that all clones 20-1A, 24-6G and 34-6E of APP669-x N-terminal-recognizing antibody reacted with APP669-711. On the other hand, no reactivity with Aβ1-40 and APP668-677 was confirmed. The sequence of APP669-711 is the same as the sequence of Aβ1-40, except that APP669-711 has longer 3 amino acids in the N-terminal side compared to the N-terminal of Aβ1-40. Nevertheless, no reactivity with Aβ1-40 was confirmed. Considering the results, it is indicated that clones 20-1A, 24-6G and 34-6E recognize the 3 amino acids in the N-terminal side of APP669-711. Furthermore, APP668-677 has an only longer 1 amino acid in the N-terminal side compared to the N-terminal of APP669-711. Nevertheless, clones 20-1A, 24-6G and 34-6E did not react with APP668-677. Considering the results, it is indicated that clones 20-1A, 24-6G and 34-6E specifically recognize the N-terminal of APP669-711.

From the above results, it was confirmed that the three clones of APP669-x N-terminal-recognizing antibody specifically recognize the N-terminal of APP669-711.

**[Table 1]**

| **SEQ ID NO.** | **Peptide Name** | **Sequence** |
|---|---|---|
| **1** | **Aβ1-40** | DAEFRHDSGYEVHHQKLVFFAEDVGSNKGAIIGLMVGGVV |
| **2** | **Aβ1-42** | DAEFRHDSGYEVHHQKLVFFAEDVGSNKGAIIGLMVGGVVIA |
| **3** | **APP669-711** | VKMDAEFRHDSGYEVHHQKLVFFAEDVGSNKGAIIGLMVGGVV |
| **4** | **APP668-677** | EVKMDAEFRH |

### [Example 2: Sandwich ELISA for APP669-711]

### [Example 2-1: Sandwich ELISA using the three clones of APP669-x N-terminal-recognizing antibody]

Reactivity of a sandwich ELISA for APP669-711 was evaluated by using APP669-x N-terminal-recognizing antibody clone 20-1A, 24-6G or 34-6E as a capture antibody.

An APP669-x N-terminal-recognizing antibody was diluted to a concentration of 20 µg/mL by a sodium carbonate buffer (pH 9.6), and was added to each well of a 96 well microplate in an amount of 50 µL, and incubated at 4 °C for 6 hours to conduct an immobilization. The solution in the plate was removed, and a 20 % Blocking One (Nacalai Tesque) was added to each well in an amount of 100 µL, and incubated at 4 °C for 2 hours to conduct a blocking. The solution in the plate was removed, and washing was conducted with 300 µL of PBST. The synthetic peptide APP669-711 that was diluted by a buffer A for sample (5 % Blocking One in PBST), as a sample solution, was added to each well in an amount of 50 µL, and incubated at 4 °C for overnight. The sample solution in the plate was removed, and washing was conducted with 300 µL of PBST. A solution of HRP-labeled C-terminal-recognizing antibody (clone BA27) contained in the Human βAmyloid (1-40) ELISA Kit (Wako Pure Chemical Corporation) was diluted five times by a 5 % Blocking One in PBST, and the resulting diluted solution was added to each well in an amount of 50 µL, and incubated at 4 °C for 2 hours. The solution in the plate was removed, and washing was conducted with 300 µL of PBST. An ELISA POD Substrate TMB Kit was added to each well in an amount of 100 µL, and an incubation in the dark for 30 minutes was conducted to develop a color. 100 µL of 2N sulfuric acid was added to stop the reaction of the color development. The absorbance of main wavelength 450nm / sub wavelength 650nm was measured by the microplate reader.

These results are shown in Fig. 4. That is, Fig. 4 is a graph showing results of a sandwich ELISA for APP669-711 using three clones of the antibodies specific to the N-terminal of APP669-x, respectively. The horizontal axis indicates APP669-711 concentration (pmol/mL), and the vertical axis indicates absorbance of main wavelength 450nm / sub wavelength 650nm measured by the microplate reader.

From Fig. 4, it was indicated that the clone 34-6E has the highest reactivity with APP669-711 among the three clones of APP669-x N-terminal-recognizing antibody. Accordingly, in the Examples hereafter, a sandwich ELISA was evaluated by focusing on 34-6E.

### [Example 2-2: Confirmation of specificity of sandwich ELISA]

Specificity of a sandwich ELISA for APP669-711 was evaluated by using APP669-x N-terminal-recognizing antibody clone 34-6E as a capture antibody. The sandwich ELISA was performed as follows.

The APP669-x N-terminal-recognizing antibody clone 34-6E was diluted to a concentration of 20 µg/mL by a sodium carbonate buffer (pH 9.6), and was added to each well of a 96 well microplate in an amount of 50 µL, and incubated at 4 °C for 6 hours to conduct an immobilization. The solution in the plate was removed, and a 4-fold dilution of Block Ace was added to each well in an amount of 100 µL, and incubated at 4 °C for 2 hours to conduct a blocking. The solution in the plate was removed, and washing was conducted with 300 µL of PBST. The synthetic peptide APP669-711 or Aβ1-40 that was diluted by a buffer B for sample [(10-fold dilution of Block Ace, 60 ng/mL anti-Aβ antibody clone 4G8 (BioLegend, epitope Aβ18-22)], as a sample solution, was added to each well in an amount of 50 µL, and incubated at 4 °C for overnight. Duplicate measurements were conducted for each sample. Further, the buffer B for sample contained the anti-Aβ antibody clone 4G8 (60 ng/mL) for the purpose of improving reactivity. The sample solution in the plate was removed, and washing was conducted with 300 µL of PBST. A solution of HRP-labeled C-terminal-recognizing antibody (clone BA27) contained in the Human βAmyloid (1-40) ELISA Kit (Wako Pure Chemical Corporation) was added to each well in an amount of 50 µL, and incubated at 4 °C for 2 hours. The solution in the plate was removed, and washing was conducted with 300 µL of PBST. An ELISA POD Substrate TMB Kit was added to each well in an amount of 100 µL, and an incubation in the dark for 30 minutes was conducted to develop a color. 100 µL of 2N sulfuric acid was added to stop the reaction of the color development. The absorbance of main wavelength 450nm / sub wavelength 650nm was measured by the microplate reader.

These results are shown in Fig. 5. That is, Fig. 5 is a graph showing specificity of a sandwich ELISA for APP669-711 in case using clone 34-6E of the antibody specific to the N-terminal of APP669-x. The horizontal axis indicates peptide concentration (pmol/mL), and the vertical axis indicates absorbance of main wavelength 450nm / sub wavelength 650nm measured by the microplate reader.

From Fig. 5, in the sandwich ELISA using the APP669-x N-terminal-specific antibody clone 34-6E and C-terminal-recognizing antibody (clone BA27), the reaction with APP669-711 was observed, however, no reaction with Aβ1-40 was observed. From the above results, the specificity of the sandwich ELISA for APP669-711 could be confirmed.

Furthermore, the synthetic peptide APP669-711 was measured in various concentrations in n=4, and a lower limit of quantitation (Blank absorbance + 2SD) was calculated to obtain a value of 2.42 fmol/mL.

### [Example 2-3: Spike and recovery test of ELISA for APP669-711]

In order to confirm an influence by measurement interfering substances in a human plasma on a measurement value, a spike and recovery test of ELISA for APP669-711 was conducted. An operation of the sandwich ELISA was conducted as the same in the operation of the above described Example 2-2. As a sample, a commercially available plasma (Tennessee Blood Services) that was diluted four times by a buffer for sample was used. APP669-711 was added such that a concentration of the added APP669-711 was 0, 5, 10, or 20 fmol/mL.

A recovery rate was evaluated, and as the results, it was indicated that the each recovery rate was in the range of 89-91 %, the range having no problems (Table 2).

**[Table 2]**

| Spiked APP669-711 Conc. (fmol/mL) | Measured Value (fmol/mL) | Theoretical Value (fmol/mL) | Recovery rate |
|---|---|---|---|
| 0 | 3.68 | | |
| 5 | 7.73 | 8.68 | 89% |
| 10 | 12.47 | 13.68 | 91% |
| 20 | 21.09 | 23.68 | 89% |

### [Example 2-4: Plasma measurement by ELISA for APP669-711]

An endogenous APP669-711 in a plasma was quantified by APP669-711 ELISA. As a sample, each of five commercially available plasma samples that was diluted four times by a buffer for sample was used, and duplicate measurements were conducted for each sample.

These results are shown in Fig. 6. That is, Fig. 6 is a graph showing results of measuring a human plasma by a sandwich ELISA for APP669-711. The horizontal axis indicates each plasma sample, and the vertical axis indicates APP669-711 concentration (fmol/mL).

From Fig. 6, any of five samples indicated the each concentration value over the lower limit of quantitation (2.42 fmol/mL). From the above results, it was indicated that the present sandwich ELISA is a sufficient measurement method for quantifying APP669-711 in a plasma.

### [Experimental Example B1: Operation method of sandwich ELISA for APP669-711]

First, the Experimental Example B1 shows a basic operation method of a sandwich ELISA for APP669-711 as follows . Each operation in Examples B1 to B4 was conducted according to this basic operation.

### (N-terminal-recognizing antibody)

Preparation of an antibody that recognizes an N-terminal of APP669-711 was asked of ITM Co., Ltd., and three clones (20-1A, 24-6G and 34-6E) were obtained

### (Immobilization of N-terminal-recognizing antibody and blocking)

N-terminal-recognizing antibody was diluted to a concentration of 20 µg/mL by a sodium carbonate buffer (pH 9.6). And, the obtained diluted solution of the antibody was added to each well of a 96 well microplate in an amount of 50 µL, and incubated at 4 °C for 2 hours to conduct an immobilization of the N-terminal-recognizing antibody. The diluted solution of the antibody in the plate was removed, and a 20 % Blocking One (Nacalai Tesque) was added to each well in an amount of 100 µL, and incubated at 4 °C for 2 hours to conduct a blocking.

### (Preparation of sample solution)

The peptide APP669-711 to be analyzed was adjusted in a predetermined concentration by a 5 % Blocking One in PBST to prepare a sample solution. Furthermore, an antibody for use as an antigen affinity substance was adjusted in an arbitrary concentration by a 5 % Blocking One in PBST. The resulting solution of the antigen affinity substance was added to the sample solution in an equal amount.

### (Addition of sample solution)

The 20 % Blocking One in the plate was removed, and washing operation with 300 µL of PBST was conducted three times. The sample solution was added to each well of the plate in an amount of 50 µL, and incubated at 4 °C for 1 hour.

### (Addition of HRP-labeled antibody recognizing C-terminal)

A solution of HRP-labeled antibody recognizing C-terminal (clone BA27) contained in the Human βAmyloid (1-40) ELISA Kit (Wako Pure Chemical Corporation) was diluted five times by a 5 % Blocking One in PBST. The sample solution in the plate was removed, and washing operation with 300 µL of PBST was conducted three times. The HRP-labeled antibody (BA27) solution that was diluted five times was added to each well in an amount of 50 µL, and incubated at 4 °C for 1 hour. The solution in the plate was removed, and washing operation with 300 µL of PBST was conducted five times.

### (Addition of enzyme-labeled antibody and measurement of absorbance)

An ELISA POD Substrate TMB Kit (Nacalai) was added to each well in an amount of 100 µL, and an incubation in the dark for 15 minutes was conducted to develop a color. 100 µL of 2N sulfuric acid was added to stop the reaction of the color development. The absorbance of main wavelength 450nm / sub wavelength 650nm was measured by a microplate reader.

### [Example B1]

### [Reactivity evaluation of sandwich ELISA for APP669-711 by addition of anti-Aβ antibody 4G8]

The Amyloid β (Aβ) 1-40 takes a structural conformation where the C-terminal and the N-terminal are near to each other in a space distance (Non-Patent Document 4). It is considered that the C-terminal and the N-terminal of APP669-711 are also near to each other, since APP669-711 has the same sequence as Aβ1-40 except that APP669-711 has longer 3 amino acids in the N-terminal side compared to the N-terminal of Aβ1-40. Accordingly, it was verified if reactivity of a sandwich ELISA is improved by adding an antibody capable of binding to APP669-711 to a sample to change the conformation of APP669-711. The anti-Aβ antibody 4G8 was used as an antibody capable of binding to APP669-711 (antigen affinity substance).

The anti-Aβ antibody 4G8 as the antigen affinity substance was added to a sample solution of APP669-711 such that a concentration of APP669-711 was 0 fmol/mL, 15.625 fmol/mL, 31.25 fmol/mL, 62.5 fmol/mL, 125 fmol/mL, 250 fmol/mL, 500 fmol/mL, or 1,000 fmol/mL, and a concentration of the anti-Aβ antibody 4G8 was 0, 0.3, 1, 3, 10, 30, 100, 300, 1,000, 3,000, or 10,000 ng/mL. Thus, sample solutions were prepared. The obtained sample solutions were measured by the sandwich ELISA for APP669-711. The epitope of the clone 4G8 is Aβ18-22. The N-terminal-recognizing antibody clone 34-6E was used as the immobilized antibody. As the results, the absorbance became higher by adding the anti-Aβ antibody 4G8 compared to no addition (0 ng/mL) of the 4G8 (Fig. 7). The absorbance became higher in a concentration dependent manner up to the 4G8 addition concentration of 100 ng/mL, however, the absorbance became gradually lower over the 4G8 addition concentration of 100 ng/mL (Fig. 8).

Regarding a reason for the improvement of the absorbance, there seems to be a possibility that the anti-Aβ antibody 4G8 was immobilized on the plate, the immobilized anti-Aβ antibody 4G8 captured APP669-711, and the captured APP669-711 were reactively sandwiched with the HRP-labeled C-terminal-recognizing antibody. In order to test this matter, by using sample solutions of Aβ1-40 (0 to 1,000 fmol/mL) that reacts with the anti-Aβ antibody 4G8, but does not react with the N-terminal-recognizing antibody 34-6E, a sandwich ELISA reactivity for APP669-711 in case of adding the anti-Aβ antibody 4G8 in a concentration of 10,000 ng/mL was evaluated. Since the epitope of the anti-Aβ antibody 4G8 is Aβ18-22, if the anti-Aβ antibody 4G8 was immobilized on the plate, then, reactivity should be indicated. However, no reaction was indicated (Fig. 9). That is, it was confirmed that the reason for the improvement of the reactivity is not that the anti-Aβ antibody 4G8 was immobilized, and the immobilized anti-Aβ antibody 4G8 captured APP669-711. From the above results, it was found that reactivity of a sandwich ELISA can be improved while maintaining the specificity for APP669-711 by adding the anti-Aβ antibody 4G8 into the sample solution.

Fig. 7 is a graph showing calibration curves of ELISA for APP669-711 by adding anti-Aβ antibody 4G8. The horizontal axis indicates APP669-711 concentration (fmol/mL), and the vertical axis indicates absorbance of 450nm/650nm. The calibration curves are represented with respect to each concentration of anti-Aβ antibody 4G8.

Fig. 8 is a graph showing reactivity of ELISA for APP669-711 by adding anti-Aβ antibody 4G8. The horizontal axis indicates concentration (ng/mL) of anti-Aβ antibody 4G8, and the vertical axis indicates absorbance of 450nm/650nm. The points are connected by a line with respect to each concentration of APP669-711.

Fig. 9 is a graph showing reactivity of ELISA for APP669-711 against Aβ1-40 by adding anti-Aβ antibody 4G8. The horizontal axis indicates concentration (fmol/mL) of Aβ1-40, and the vertical axis indicates absorbance of 450nm/650nm. An addition amount of the anti-Aβ antibody 4G8 was 10,000 ng/mL.

### [Example B2: Verification of addition effect of anti-Aβ antibody 4G8 in the case of changing clone of N-terminal-recognizing antibody]

Experiments were conducted in order to verify if addition effect of anti-Aβ antibody 4G8 is obtained, in the case of changing clone of the N-terminal-recognizing antibody used as the immobilized antibody.

The anti-Aβ antibody 4G8 as the antigen affinity substance was added to a sample solution of APP669-711 such that a concentration of APP669-711 was 500 fmol/mL, and a concentration of the anti-Aβ antibody 4G8 was 0, 10, 30, 100, 300, 1,000, or 3,000 ng/mL. Thus, sample solutions were prepared. The obtained sample solutions were measured by the sandwich ELISA for APP669-711. The three clones 20-1A, 24-6G, and 34-6E of the N-terminal-recognizing antibody were used, respectively, as the immobilized antibody. As the results, it was confirmed that the absorbance increased in all three clones, and the addition concentration of the anti-Aβ antibody 4G8 indicating the highest absorbance was 100 ng/mL in common in respective clones (Fig. 10). That is, in any clones used, the most suitable addition concentration of the anti-Aβ antibody 4G8 was the same.

Furthermore, in the sandwich ELISA for APP669-711 wherein the each clone was immobilized, a reactivity of sample solutions of Aβ1-40 (0 to 1,000 fmol/mL) in case of adding the anti-Aβ antibody 4G8 in a concentration of 3,000 ng/mL was evaluated. As the results, no reaction was observed (Fig. 11). That is, it was confirmed that the reason for the improvement of the reactivity is not that the anti-Aβ antibody 4G8 was immobilized, and the immobilized anti-Aβ antibody 4G8 captured APP669-711.

Fig. 10 is a graph showing adding effect of anti-Aβ antibody 4G8 in ELISA for APP669-711 using three clones of the antibodies recognizing the N-terminal, respectively. The horizontal axis indicates concentration (ng/mL) of anti-Aβ antibody 4G8, and the vertical axis indicates absorbance of 450nm/650nm. The points are connected by a line with respect to each clone of the antibody recognizing the N-terminal.

Fig. 11 is a graph showing reactivity of ELISA for APP669-711 against Aβ1-40 by adding anti-Aβ antibody 4G8. The three clones of the antibodies recognizing the N-terminal were used, respectively. The horizontal axis indicates concentration (fmol/mL) of Aβ1-40, and the vertical axis indicates absorbance of 450nm/650nm. An addition amount of the anti-Aβ antibody 4G8 was 3,000 ng/mL.

### [Example B3: Verification of reactivity improvement effect of sandwich ELISA for APP669-711 in the case of changing anti-Aβ antibody to be added]

Experiments were conducted in order to verify if reactivity improvement effect of the sandwich ELISA for APP669-711 is obtained, in the case of changing clone of the anti-Aβ antibody to be added to the sample.

Each of four clones (4G8, 6E10, BAM90.1, or NAB228) of the anti-Aβ antibody as the antigen affinity substance was added to a sample solution of APP669-711 such that a concentration of APP669-711 was 500 fmol/mL, and a concentration of the anti-Aβ antibody 4G8 was 0, 10, 30, 100, 300, 1,000, or 3,000 ng/mL. Thus, sample solutions were prepared. The obtained sample solutions were measured by the sandwich ELISA for APP669-711. The epitope of the clone 6E10 is Aβ3-8, the epitope of the clone BAM90.1 is Aβ20-23, and the epitope of the clone NAB228 is a portion included in Aβ1-11. The N-terminal-recognizing antibody clone 34-6E was used as the immobilized antibody. As the measurement results, it was observed that the absorbance increased in all four clones of the anti-Aβ antibodies that were added (Fig. 12). The concentration value indicating the highest absorbance in the each clone was as follows;
100 ng/mL in case of the clone 4G8,
300 ng/mL in case of the clone 6E10,
1,000 ng/mL in case of the clone BAM90.1, and
3,000 ng/mL in case of the clone NAB228.
That is, the most suitable concentration value was different depending on the clones.

Furthermore, in the sandwich ELISA for APP669-711, a reactivity of sample solutions of Aβ1-40 (0 to 1,000 fmol/mL, specifically, 0 fmol/mL, 500 fmol/mL, or 1,000 fmol/mL) in case of adding the each clone of the anti-Aβ antibody in a concentration of 3,000 ng/mL was evaluated. As the results, no reaction was observed (Fig. 13). That is, it was confirmed that the reason for the improvement of the reactivity is not that the each clone of the anti-Aβ antibody was immobilized in the case of adding any clone, and the immobilized each clone of anti-Aβ antibody captured APP669-711.

Fig. 12 is a graph showing adding effect of four clones of anti-Aβ antibodies in ELISA for APP669-711, respectively. The horizontal axis indicates concentration (ng/mL) of anti-Aβ antibodies, and the vertical axis indicates absorbance of 450nm/650nm. The points are connected by a line with respect to each clone of the anti-Aβ antibody.

Fig. 13 is a graph showing reactivity of ELISA for APP669-711 against Aβ1-40 by adding anti-Aβ antibody. The four clones of the anti-Aβ antibodies were used, respectively. The horizontal axis indicates concentration (fmol/mL) of Aβ1-40, and the vertical axis indicates absorbance of 450nm/650nm. An addition amount of the anti-Aβ antibody was 3,000 ng/mL.

### [Example B4: Verification of addition effect of anti-Aβ antibody in sandwich ELISA for Aβ1-40]

Experiments were conducted in order to verify if reactivity improvement of the sandwich ELISA for Aβ1-40 is also obtained by adding an anti-Aβ antibody, with the use of the Human βAmyloid (1-40) ELISA Kit (Wako Pure Chemical Corporation).

As a sample, the "standard" contained in the Human βAmyloid (1-40) ELISA Kit or Aβ1-40 purchased from AnaSpec was used. The anti-Aβ antibody clone 4G8 or 6E10 was added to each Aβ1-40 sample ("Standard in kit" or "AnaSpec product") such that a concentration of Aβ1-40 was 50 fmol/mL, and a concentration of the 4G8 was 100 ng/mL or a concentration of the 6E10 was 300 ng/mL. Thus, sample solutions were prepared. The obtained sample solutions were measured to obtain absorbance by performing the operations according to the protocol in the instruction manual of the Human βAmyloid (1-40) ELISA Kit. Furthermore, as a control, the same operations were performed except that both the 4G8 and the 6E10 were not added, to obtain a measured absorbance (in Fig. 14, referred to as "non-spiked") . As the results, it was observed that the absorbance increased in case of adding the clone 4G8 compared to the "non-spiked", however, there was no change in absorbance in case of adding the clone 6E10 compared to the "non-spiked". The epitope of the 6E10 is Aβ3-8, that is near to the N-terminal of Aβ1-40. Therefore, it is considered that the N-terminal-recognizing antibody that was used as the immobilized antibody in ELISA inhibits the binding formation with the 6E10 in a steric structure. On the other hand, the epitope of the clone 4G8 is Aβ18-22, that is kept away from the N-terminal-recognizing antibody in a space distance. Therefore, it is considered that the clone 4G8 contributed to improve the reactivity of the sandwich ELISA for Aβ1-40.

For this reason, regarding an anti-Aβ antibody to be added as an antigen affinity substance, it is considered that one should select an antibody whose epitope exists in a portion that is kept away in some space distance from an epitope to be recognized by an antibody used in a sandwich ELISA.

Fig. 14 is a graph showing adding effect of anti-Aβ antibodies 4G8 and 6E10, respectively, in ELISA for Aβ1-40. The vertical axis indicates absorbance of 450nm/650nm. As the samples, the standard contained in the ELISA kit, and Aβ1-40 available from AnaSpec were used, respectively.

From the results of Examples B1 to B4, it was found that by adding an antibody capable of binding to a polypeptide to be analyzed, such as APP669-711 and Aβ1-40, the reactivity improvement effect of the sandwich ELISA for each polypeptide is obtained.

In the above described respective Examples, the cases where a polypeptide to be analyzed is APP669-711 and Aβ1-40 were demonstrated. The present invention is also useful for a sandwich ELISA where a polypeptide or a protein other than these polypeptides is to be analyzed. Furthermore, the present invention is also applicable to a sandwich analysis method using other labeling as well as ELISA method.

## Claims

1. An APP669-x N-terminal-recognizing monoclonal antibody that specifically recognizes an N-terminal of an APP669-x peptide.

2. An immunoassay method that uses an APP669-x N-terminal-recognizing monoclonal antibody that specifically recognizes an N-terminal of an APP669-x peptide, and comprises reacting an APP669-x in a sample with the APP669-x N-terminal-recognizing monoclonal antibody to measure the APP669-x.

3. The immunoassay method according to claim 2, wherein the method is selected from the group consisting of a sandwich immunoassay method, a direct ELISA, an indirect ELISA, a competitive ELISA, a western blotting, an immunohistochemistry, a flow cytometry, an immunoprecipitation, an affinity chromatography, and an immunocytochemistry.

4. The immunoassay method according to claim 3, wherein the method uses, in the sandwich immunoassay method,
the APP669-x N-terminal-recognizing monoclonal antibody, as a first antibody; and
an anti-Aβ antibody, as a second antibody, capable of recognizing a portion of the APP669-x that is different from the antigen binding site of the first antibody, or a C-terminal of a fragment of the APP669-x.

5. The immunoassay method according to claim 3, wherein the second antibody is a monoclonal antibody or a polyclonal antibody that is capable of recognizing a C-terminal of APP669-711.

6. The immunoassay method according to claim 3, wherein the sandwich immunoassay method is selected from the group consisting of an enzyme immunoassay method (EIA), a radio immunoassay method (RIA), a chemiluminescent immunoassay method (CIA), a fluorescent immunoassay method (FIA), an electro-chemiluminescent immunoassay method (ECLIA), a bioluminescent immunoassay method (BLIA), an immuno-PCR, a turbidimetric immunoassay method (TAI), and a latex agglutination turbidimetric method (LA).

7. The immunoassay method according to claim 3, wherein the immunoassay method comprises adding an antigen affinity substance capable of binding to the APP669-x to the sample.

8. The immunoassay method according to claim 7, wherein the antigen affinity substance is added to the sample, and then, the APP669-x peptide is reacted with the first antibody and the second antibody; or
while the APP669-x peptide is reacted with the first antibody and the second antibody, the antigen affinity substance is added to a reaction system; or
the APP669-x peptide is reacted with either one of the first antibody and the second antibody, and then, the antigen affinity substance is added to a reaction system, and then, the APP669-x peptide is reacted with the other one of the first antibody and the second antibody.

9. The immunoassay method according to claim 7, wherein the antigen affinity substance is selected from the group consisting of an antibody, a peptide, a low molecule compound, and a nucleic acid aptamer.

10. The immunoassay method according to claim 2, wherein the sample is a living body-derived sample selected from the group consisting of blood, cerebrospinal fluid, urine, feces, and body secreting fluid.

11. A kit for sandwich immunoassay method for APP669-711, comprising:
an APP669-x N-terminal-recognizing monoclonal antibody, as a first antibody, that specifically recognizes an N-terminal of an APP669-x peptide; and
a monoclonal antibody or a polyclonal antibody, as a second antibody, that is capable of recognizing a C-terminal of APP669-711.

12. A kit for sandwich immunoassay method for APP669-711 according to claim 11, further comprising an antigen affinity substance capable of binding to the APP669-x.

13. A sandwich immunoassay method for a polypeptide, which uses a first antibody having an antigen binding site capable of recognizing a target polypeptide in a sample, and a second antibody having an antigen binding site that is different from the antigen binding site of the first antibody and is capable of recognizing the target polypeptide,
wherein the immunoassay method comprises adding an antigen affinity substance capable of binding to the target polypeptide to the sample.

14. The immunoassay method according to claim 13, wherein the antigen affinity substance is added to the sample containing the target polypeptide, and then, the target polypeptide is reacted with the first antibody and the second antibody; or
while the target polypeptide is reacted with the first antibody and the second antibody, the antigen affinity substance is added to a reaction system; or
the target polypeptide is reacted with either one of the first antibody and the second antibody, and then, the antigen affinity substance is added to a reaction system, and then, the target polypeptide is reacted with the other one of the first antibody and the second antibody.

15. The immunoassay method according to claim 13, wherein the antigen affinity substance is selected from the group consisting of an antibody, a peptide, a low molecule compound, and a nucleic acid aptamer.

16. The immunoassay method according to claim 13, wherein sandwich immunoassay method is selected from the group consisting of an enzyme immunoassay method (EIA), a radio immunoassay method (RIA), a chemiluminescent immunoassay method (CIA), a fluorescent immunoassay method (FIA), an electro-chemiluminescent immunoassay method (ECLIA), a bioluminescent immunoassay method (BLIA), an immuno-PCR, a turbidimetric immunoassay method (TAI), and a latex agglutination turbidimetric method (LA).

17. The immunoassay method according to claim 13, wherein the first antibody is an antibody that recognizes an N-terminal of the target polypeptide, and the second antibody is an antibody that recognizes a C-terminal of the target polypeptide.

18. The immunoassay method according to claim 17, wherein the antigen affinity substance is a substance that does not act on a neighborhood of the N-terminal of the target polypeptide, and does not act on a neighborhood of the C-terminal of the target polypeptide.

19. The immunoassay method according to claim 17, wherein the antigen affinity substance is a substance that act on an intermediate portion comprising a portion from a fourth residue site from the N-terminal of the target polypeptide, to a fourth residue site from the C-terminal of the target polypeptide.

20. The immunoassay method according to claim 13, wherein the target polypeptide is selected from the group consisting of an Aβ and an Aβ related peptide.

21. The immunoassay method according to claim 13, wherein the sample is a living body-derived sample selected from the group consisting of blood, cerebrospinal fluid, urine, feces, and body secreting fluid.

22. A kit for sandwich immunoassay method for a polypeptide, comprising:
a first antibody having an antigen binding site capable of recognizing a target polypeptide;
a second antibody having an antigen binding site that is different from the antigen binding site of the first antibody and is capable of recognizing the target polypeptide; and
an antigen affinity substance capable of binding to the target polypeptide.
